Europäisches Patentamt

European Patent Office

Office européen des brevets

(19)

(11) Publication number: **0 118 850**

**B1**

(12) **EUROPEAN PATENT SPECIFICATION**

(45) Date of publication of patent specification: **23.12.87**

(51) Int. Cl.⁴: **A 61 M 16/00**

(21) Application number: **84102241.1**

(22) Date of filing: **02.03.84**

(54) **Apparatus for independent ventilation of two lungs with selective use of positive end-expiratory pressures.**

(30) Priority: **04.03.83 PL 240878**

(43) Date of publication of application:
**19.09.84 Bulletin 84/38**

(45) Publication of the grant of the patent:
**23.12.87 Bulletin 87/52**

(84) Designated Contracting States:
**DE FR GB IT NL SE**

(56) References cited:
**US-A-3 762 427**
**US-A-3 923 056**

**IEEE TRANSACTIONS ON BIOMEDICAL ENGINEERING, vol. BME-29, no. 11, November 1982, pages 736-740, IEEE, New York, US; T.D. EAST et al.: "A microcomputer-based differential lung ventilation system"**

(73) Proprietor: **Polska Akademia Nauk Instytut Biocybernetyki i Inzynierii Biomedycznej ul.Krajowej Rady Narodowej 55 Warszawa (PL)**

(72) Inventor: **Darowski, Marek ul. Korotynskiego 48 m. 187 Warszawa (PL)**
Inventor: **Hedenstierna, Göran Vendevägen 74 18264 Djursholm (SE)**

(74) Representative: **Ebbinghaus, Dieter et al v. FÜNER, EBBINGHAUS, FINCK Patentanwälte European Patent Attorneys Mariahilfplatz 2 & 3 D-8000 München 90 (DE)**

Courier Press, Leamington Spa, England.

### Description

This invention relates to an apparatus for independent ventilation of two lungs with selective use of positive end-expiratory pressures.

Independent ventilation of two lungs with selective use of positive end-expiratory pressures consists in selection of inspiratory volumes and positive end-expiratory pressures for each of lungs in such a way that their ventilation will be adapted to their perfusion. This method of artificial ventilation ensures better, more effective gas exchange in the lungs during anesthesia than in the case of non-controlled division of inspiratory volume between lungs and use of equal values of positive end-expiratory pressures for both lungs.

A known apparatus for independent ventilation of two lungs with selective use of positive expiratory pressures contains generally two synchronized respirators coupled with lungs by means of a double-lumen tracheal tube, through inspiratory and expiratory branches. Those branches are connected together in two inspiratory-expiratory pairs, each comprising an inspiratory and an expiratory branch terminating in a common point connected with the tracheal tube. One inspiratory-expiratory pair is connected with one of two conduits of this tube, while a second pair is connected with the other conduit of the tracheal tube.

The apparatus for independent ventilation of two lungs with selective use of positive end-expiratory pressures according to the invention comprises only one respirator connected to the double-lumen tracheal tube through the monitoring-controlling means containing two parallel inspiratory paths and two parallel expiratory paths. Each path is coupled with the double-lumen tracheal tube respectively through the inspiratory and expiratory branches. In one inspiratory path having a common point with the expiratory path not provided with positive end-expiratory pressure valve is placed the threshold inspiratory valve connected in series with a pneumatic unidirectional valve.

A pneumatic unidirectional valve is situated as well in the inspiratory path having a common point with the expiratory path provided with the positive end-expiratory pressure valve.

In case of need there is placed in each inspiratory path a pneumatic resistor which resistors are interconnected in parallel.

In another embodiment there is placed in each of the inspiratory paths a threshold inspiratory valves. In each inspiratory path the threshold inspiratory valve is connected in series with the pneumatic unidirectional valve. In each expiratory path there is placed a positive end-expiratory pressure valve.

Moreover, in each inspiratory path there is placed in case of need a pneumatic resistor which resistors are interconnected in parallel.

The apparatus being the object of invention eliminates the necessity of use of two respirators for the realization of independent ventilation of two lungs with the use of positive end-expiratory pressures. Thus, the ventilation is realized using only one respirator.

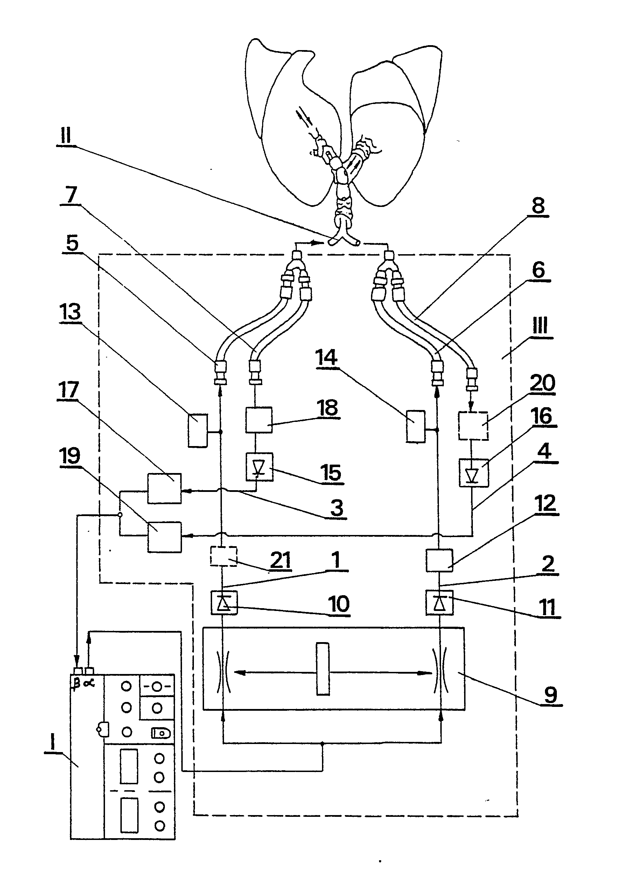

The invention will be explained in detail by way of a preferred embodiment shown in the drawing displaying the diagram of an apparatus for independent ventilation of two lungs with selective use of positive end-expiratory pressures.

The apparatus consists of a respirator I, coupled with the lungs by means of double-lumen tracheal tube II through the monitoring-controlling means III. The monitoring-controlling means III contains two parallel inspiratory paths 1 and 2, as well as two parallel expiratory paths 3 and 4. The inspiratory and expiratory paths terminate in respective inspiratory branches 5 and 6 as well as in expiratory 7 and 8 branches, connected in inspiratory-expiratory pairs. In each pair there are interconnected the inspiratory branch 5 with the expiratory branch 7 and the inspiratory branch 6 with the expiratory branch 8. The common point of the inspiratory branch 5 and the expiratory branch 7 is coupled with one of two conduits of the double-lumen tracheal tube II, while the common point of the inspiratory branch 6 and the expiratory branch 8 is coupled with the second conduit of the tracheal tube II.

In the parallel inspiratory paths 1 and 2 there are placed the coupled pneumatic resistors 9. The pneumatic resistor 9 in the inspiratory path 1 is connected in series with a pneumatic unidirectional valve 10 and the inspiratory branch 5, while pneumatic resistor 9 in the inspiratory path 2 is connected in series with a pneumatic unidirectional valve 11, a threshold inspiratory valve 12 and the inspiratory branch 6. In the inspiratory paths 1 and 2 there are placed pressure gauges 13 and 14. In the parallel expiratory paths 3 and 4 there are placed pneumatic unidirectional valves 15 and 16. The unidirectional valve 15 is connected in series with a volume meter 17, a positive end-expiratory pressure valve (PEEP) 18 and the expiratory branch 7, while the unidirectional valve 16 is connected in series with the volume meter 19 and the expiratory branch 8.

The common input of the coupled pneumatic resistors 9 is connected with the inspiratory port α of the respirator, while the common output of the volume meters 17 and 19 is connected with the expiratory port β of the respirator.

The apparatus according to the invention operates as follows. In the inspiratory phase gas outflowing from the inspiratory port α of the respirator is divided between two inspiratory paths 1 and 2 and flows through the coupled pneumatic resistors 9 and the pneumatic unidirectional valves 10 and 11. Gas flowing through inspiratory path 1 is directed by means of the inspiratory branch 5 and one of the conduits of the tracheal tube to one lung, as during the inspiratory phase the expiratory port β of the respirator is closed. Gas flowing through the inspiratory path 2 and the threshold inspiratory valve 12 is directed by means of the inspiratory branch 6 and the second conduit of the tracheal tube II to the other lung. The increase of pressure in the inspiratory branches 5 and 6 during the inspiratory phase is measured and indicated by the pressure gauges 13 and 14, respectively. In the expiratory phase the inspiratory port α of the respirator is closed, while the expiratory port β of the respirator is open, resulting in an outflow of gases from the lungs. Thus, from the one lung the gas flows through one of the conduits of the tracheal tube II via the expiratory branch 7, the positive end-expiratory pressure valve (PEEP) 18, the pneumatic unidirectional valve 15, and the volume meter 17 down to expiratory port β of the respirator. On the other hand, from the second lung the gas flows through the second conduit of the tracheal tube II via the expiratory branch 8, the pneumatic unidirectional valve 16 and the volume meter 19 down to expiratory port β of the respirator. The pneumatic unidirectional valves 10, 11, 15, 16 placed in definite places of the examined system of connections of the apapratus according to the invention determine the above described flow of gases between the respirator and the lungs in individual phases of the end-respiratory cycle (i.e. during the inspiratory and expiratory phases), enabling in this way both the realization of indpendent ventilation of each lung and establishing positive end-expiratory pressure in one of the lungs only (by means of PPEP 18 valve). The threshold inspiratory valve 12 placed in the inspiratory path 2 of the second lung has to compensate the change of gas flow rate during the inspiratory phase in the inspiratory path 1, occurring after introduction of positive end-expiratory pressure by means of the PEEP valve 18, or during dynamic changes of the values of mechanical parameters of the patient's respiratory system. By means of the coupled pneumatic resistors 9 at the same time the gas flow is being diminished in one inspiratory path, and increased in the other one.

In another embodiment the apparatus according to the invention is additionally provided with a positive end-expiratory pressure valve PEEP 20, used for setting the positive end-expiratory pressure in the expiratory branch 8 (and therefore as well in the lung coupled with this branch through the conduit of the tracheal tube), and provided as well with a threshold inspiratory valve 21, compensating the changes of gas flow rate during the inspiratory phase in the inspiratory path 2.

The above embodiment apart from the possibility of independent ventilation of each lung enables as well the establishing of positive end-expiratory pressures independently in both lungs.

## Claims

1. An apparatus for the independent ventilation of two lungs with selective use of positive end-expiratory pressures, consisting of a respirator (I) coupled with the lungs by means of a double-lumen tracheal tube (II) through two inspiratory and two expiratory branches (5, 6, 7, 8) connected in two inspiratory-expiratory pairs, wherein each pair terminates in a common point of inspiratory and expiratory branches and is coupled with one of two conduits of the double-lumen tracheal tube (II), and one of the expiratory branches contains a positive end-expiratory pressure valve (18), characterized by there being only one respirator (I) connected with the double-lumen tracheal tube (II) through monitoring-controlling means (III) containing two parallel inspiratory paths (1, 2) and two parallel expiratory paths (3, 4), each path coupled with the double-lumen tracheal tube (II) through inspiratory (5, 6) and expiratory (7, 8) branches respectively, while in the inspiratory path (2) having a common point with the expiratory path (4) not provided with the positive end-expiratory pressure valve is placed a threshold inspiratory valve (12), the inspiratory valve (12) being connected in series with a pneumatic unidirectional valve (11), there being a further pneumatic unidirectional valve (10) situated in the other inspiratory paths (1).

2. An apparatus for independent ventilation of two lungs with selective use of positive end-expiratory pressures, consisting of a respirator (I) coupled with the lungs by means of a double-lumen tracheal tube (II) through two inspiratory and two expiratory branches (5, 6, 7, 8) connected in two inspiratory-expiratory pairs, wherein each pair terminates in a common point of the inspiratory and expiratory branches and is coupled with one of two conduits of double-lumen tracheal tube (II), and one of the expiratory branches contains a positive end-expiratory pressure valve (18), characterized by there being only one respirator (I) connected with the double-lumen tracheal tube (II) through monitoring-controlling means (III) containing two parallel inspiratory paths (1, 2) and two parallel expiratory paths (3, 4), each path coupled with the double-lumen tracheal tube (II) through inspiratory (5, 6) and expiratory (7, 8) branches respectively, there being in each of the inspiratory paths (1, 2) a threshold inspiratory valve (12, 21) connected in series with a pneumatic unidirectional valve (10, 11), and there being each expiratory path (3, 4) a positive end-expiratory pressure valve (18, 20).

3. An apparatus according to claim 1 or 2, characterized in that in each inspiratory path (1, 2) is placed a pneumatic resistor (9), which resistors are interconnected in parallel.

## Patentansprüche

1. Vorrichtung zum unabhängigen Beatmen der beiden Lungenflügel mit wahlweiser Ausnutzung von Zwangsendausatmungsdrucken, bestehend aus einem Beatmungsgerät (I), das mit der Lunge mittels eines Doppellumendrachialrohrs (II) über zwei Einatmungs- und zwei Ausatmungszweigleitungen (5, 6, 7, 8) gekoppelt ist, die zu zwei Einatmungs-Ausatmungspaaren verbunden sind, wobei jedes Paar in einer gemeinsamen Weiche der Einatmungs- und Ausatmungszweigleitungen endet und mit einer der beiden Leitungen des Doppellumendrachialrohrs (II) gekoppelt ist, und wobei eine der Ausatmungsweigleitungen ein Zwangsendausatmungsdruckventil (18) enthält, dadurch gekennzeichnet, daß nur ein Beatmungsgerät (I) vorgesehen ist, das mit dem Doppellumendrachialrohr (II) über Überwachungs-Steuereinrichtungen (III) verbunden ist, die zwei parallele Einatmungswege (1, 2) und zwei parallele Ausatmungswege (3, 4) enthalten, wobei jeder Weg mit dem Doppellumendrachialrohr (II) über die Einatmungszweigleitungen (5, 6) bzw. Ausatmungszweigleitungen (7, 8) gekoppelt ist, während in dem Einatmungsweg (2), der eine gemeinsame Weiche mit dem nicht mit dem Zwangsendausatmungsdruckventil versehenen Ausatmungsweg (4) hat, ein Schwellenwerteinatmungsventil (12) angeordnet ist, das in Reihe zu einem pneumatischen unidirektionalem Ventil (11) geschaltet ist, wobei in den anderen Einatmungswegen (1) ein weiteres unidirektionales Ventil (10) angeordnet ist.

2. Vorrichtung zum unabhängigen Beatmen der beiden Lungenflügel mit wahlweiser Ausnutzung von Zwangsendausatmungsdrucken, bestehend aus einem Beatmungsgerät (I), das mit der Lunge mittels eines Doppellumendrachialrohrs (II) über zwei Einatmungs- und zwei Ausatmungszweigleitungen (5, 6, 7, 8) gekoppelt ist, die zu zwei Einatmungs-Ausatmungspaaren verbunden sind, wobei jedes Paar in einer gemeinsamen Weiche der Einatmungs- und Ausatmungszweigleitungen endet und mit einer der beiden Leitungen des Doppellumendrachialrohr (II) gekoppelt ist, und wobei eine der Ausatmungszweigleitungen ein Zwangsendausatmungsdruckventil (18) enthält, dadurch gekennzeichnet, daß nur ein Beatmungsgerät (I) vorgesehen ist, das mit dem Doppellumendrachialrohr (II) über Überwachungs-Steuereinrichtungen (III) verbunden ist, die zwei parallele Einatmungswege (1, 2) und zwei parallele Ausatmungswege (3, 4) enthalten, wobei jeder Weg mit dem Doppellumendrachialrohr (II) über die Einatmungszweigleitungen (5, 6) bzw. Ausatmungszweigleitungen (7, 8) gekoppelt ist, daß in jedem der Einatmungswege (1, 2) ein Schwellenwerteinatmungsventil (12, 21) angeordnet ist, das in Reihe zu einem pneumatischen unidirektionalen Ventil (10, 11) geschaltet ist, und daß in jedem Ausatmungsweg (3, 4) ein Zwangsendausatmungsdruckventil (18, 20) vorgesehen ist.

3. Vorrichtung nach Anspruch 1 oder 2, dadurch gekennzeichnet, daß in jedem Einatmungsweg (1, 2) ein pneumatischer Widerstand (9) angeordnet ist, wobei die Widerstände parallel miteinander verbunden sind.

**Revendications**

1. Appareil pour la respiration artificielle indépendante des deux poumons avec utilisation sélective de surpressions à la fin de l'expiration, consistant en un appareil respiratoire (I) relié aux poumons par un tube trachéal à double lumière (II) et par l'intermédiaire de deux embranchements d'inspiration et deux embranchements d'expiration (5, 6, 7, 8) reliés de façon à constituer deux paires d'inspiration-expiration, chaque paire se terminant en un point commun aux embranchements d'inspiration et d'expiration et étant branchée sur l'un de deux conduits du tube trachéal à double lumière (II), tandis que l'un des embranchements d'expiration contient une soupape de surpression de fin d'expiration (18), caractérisé en ce qu'il n'existe qu'un seul respirator (I) qui est relié au tube trachéal à double lumière (II) par l'intermédiaire de moyens de surveillance et commande (III) qui présentent deux trajets parallèles d'inspiration (1, 2) et deux trajets parallèles d'expiration (3, 4), chaque trajet étant branché sur le tube trachéal à double lumière (II) par l'intermédiaire respectivement des embranchements d'inspiration (5, 6) et d'expiration (7, 8), tandis qu'une soupape d'inspiration à seuil (12) est disposée dans le trajet d'inspiration (2) qui offre un point commun avec le trajet d'expiration (4) ne comportant pas la soupape de surpression de fin d'expiration, cette soupape d'inspiration (12) étant branchée en série avec une soupape pneumatiqe unidirectionnelle (11) et une autre valve pneumatique unidirectionnelle (10) étant disposée sur l'autre trajet d'inspiration (1).

2. Appareil pour la respiration artificielle indépendante des deux poumons avec utilisation sélective de surpressions à la fin de l'expiration, consistant en un appareil respiratoire (I) relié aux poumons par un tube trachéal à double lumière (II) et par l'intermédiaire de deux embranchements d'inspiration et deux embranchements d'expiration (5, 6, 7, 8) reliés de façon à constituer deux paires d'inspiration-expiration, chaque paire se terminant en un point commun aux embranchements d'inspiration et d'expiration et étant branchée sur l'un de deux conduits du tube trachéal à double lumière (II), tandis que l'un des embranchements d'expiration contient une soupape de surpression de fin d'expiration (18), caractérisé en ce qu'il n'existe qu'un seul respirateur (I) qui est relié au tube trachéal à double lumière (II) par l'intermédiaire de moyens de surveillance et commande (III) qui présentent deux trajets parallèles d'inspiration (1, 2) et deux trajets parallèles d'expiration (3, 4), chaque trajet étant branché sur le tube trachéal à double lumière (II) par l'intermédiaire respectivement des embranchements d'inspiration (5, 6) et d'expiration (7, 8), tandis qu'il est prévu dans chacun des trajets d'inspiration (1, 2) une soupape d'inspiration à seuil (12, 21) qui est branchée en série avec une soupape pneumatique unidirectionnelle (10, 11) et qu'il est prévu dans chaque trajet d'expiration (3, 4) une soupape de surpression de fin d'expiration (18, 20).

3. Appareil selon la revendication 1 ou 2, caractérisé en ce qu'une résistance pneumatique (9) est disposée dans chaque trajet d'inspiration (1, 2), ces résistances étant branchées en parallèle entre elles.

0 118 850

1